(19) Europäisches Patentamt
European Patent Office
Office européen des brevets

(11) Veröffentlichungsnummer: **0 001 203**
**B1**

(12) **EUROPÄISCHE PATENTSCHRIFT**

(21) Anmeldenummer: **78100446.0**

(22) Anmeldetag: **20.07.78**

(51) Int. Cl.³: **C 07 C 127/22,**
A 01 N 9/20

(54) **N-Phenyl-N'-Benzoylharnstoffe, Verfahren zu ihrer Herstellung, diese enthaltende Mittel und ihre Verwendung in der Schädlingsbekämpfung**

(30) Priorität: **28.07.77 CH 9349/77**
**29.06.78 CH 7101/78**

(43) Veröffentlichungstag der Anmeldung:
**04.04.79 Patentblatt 79/07**

(45) Bekanntmachung des Hinweises auf die Patenterteilung:
**03.09.80 Patentblatt 80/18**

(84) Benannte Vertragsstaaten: **BE CH DE FR GB NL**

(56) Entgegenhaltungen:
**Journal of Agricultural and Food Chemistry, Band 21 (1973) Nummer 3, Seiten 348—354.**

(73) Patentinhaber: **Ciba-Geigy AG**
**Patentabteilung Postfach**
**CH - 4002 Basel CH**

(72) Erfinder: **Ehrenfreund, Josef, Dr.**
**Langenhagweg 11**
**CH - 4123 Allschwil CH**

**EP 0 001 203 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

N-Phenyl-N'-Benzoylharnstoffe, Verfahren zur ihrer Herstellung, diese enthaltende Mittel und ihre Verwendung in der Schädlingsbekämpfung

Die vorliegende Erfindung betrifft neue N-Phenyl-N'-benzoylharnstoffe, Verfahren zu ihrer Herstellung und ihre Verwendung in der Schädlingsbekämpfung.

Diese N-Phenyl-N'-benzoylharnstoffe haben die Formel I

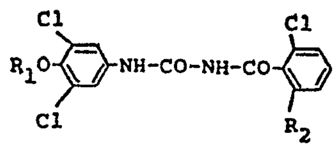

(I),

worin $R_1$ $CH_2{=}CH{-}CH_2{-}$, $CHCl{=}CCL{-}$, $CHCl{=}CH{-}CH_2{-}$, $CH_2{=}CCl{-}CH_2{-}$, $CCl_2{=}CH{-}CH_2{-}$, $CHCl{=}CCl{-}CH_2{-}$ oder $CH{\equiv}C{-}CH_2{-}$ und

$R_2$ Wasserstoff oder Chlor bedeuten.

Eine bevorzugte Gruppe von erfindungsgemässen Verbindungen der Formel I ist dadurch gekennzeichnet, dass $R_2$ Wasserstoff bedeutet.

Aufgrund ihrer Wirkung, insbesondere gegenüber Schädlingen auf dem Hygiene-Sektor, sind ferner diejenigen Verbindungen der Formel I hervorzuheben, bei denen $R_1$ den $CH_2{=}CH{-}CH_2{-}$, $CHCl{=}CCl{-}$ oder $CHCl{=}CH{-}CH_2{-}$Rest bedeutet.

Die erfindungsgemässen Verbindungen der Formel I können nach an sich bekannten Verfahren hergestellt werden, beispielsweise durch Umsetzung

a) einer Verbindung der Formel II

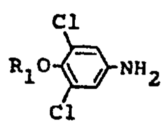

(II)

mit einer Verbindung der Formel III

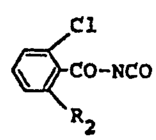

(III)

oder

b) einer Verbindung der Formel IV

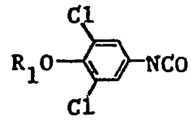

(IV)

mit einer Verbindung der Formel V

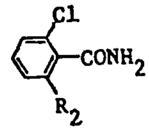

(V)

In den obigen Formeln II bis V haben $R_1$ und $R_2$ die unter Formel I angegebenen Bedeutungen.

Die erwähnten Verfahren a) und b) können unter normalem oder erhöhtem Druck und vorzugsweise in Gegenwart eines organischen Lösungs- oder Verdünnungsmittels durchgeführt werden. Als Lösungs- oder Verdünnungsmittel eignen sich z.B. Aether und ätherartige Verbindungen wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile wie Acetonitril oder Propionitril; Dimethylsulfoxid sowie Ketone, z.B. Aceton, Methyläthylketon, Methylisopropylketon und Methylisobutylketon. Verfahren a) kann bei einer Temperatur von —10 bis 100°C, vorzugsweise zwischen 15 und 25°C, gegebenenfalls in Gegenwart einer basischen Substanz, z.B. einer organische Base, wie Triäthylamin, durgeführt werden. Die Durchführung von Verfahren b) erfolgt im allgemeinen bei einer Temperatur von 0 bis 120°C, vorzugsweise beim Siedepunkt des verwendeten Lösungsmittels, und in Gegenwart einer organischen Base, wie Pyridin, und/oder unter Zusatz eines Alkali- oder Erdalkalimetalls, vorzugsweise Natrium.

Die Verbindungen der Formel I fallen — wo dies prinzipiell möglich ist — als cis/trans-Isomerengemische an. In diesem Sinne sind als Verbindungen der Formel I sowohl die cis-oder trans-Formen als auch die entsprechenden Isomeren-gemische zu verstehen. Ein Isomerengemisch kann z.B. mit Hilfe der bekannten chromatographischen Trennmethoden und anschliessender Eluierung in die isomeren Formen getrennt werden. Eine weitere chromatographische Trennmethode ist die Gaschromatographie. In gewissen Fällen kann die Isomerentrennung auch durch fraktionierte Kristalisation erfolgen.

Die Ausgangsstoffe der Formeln II, III, IV und V sind zum Teil bekannt oder können analog bekannten Verfahren hergestellt werden. Die Aniline der Formel II sind unter Anwendung der in Chem. Ber. *34*, 1940 und Ann. *418*, 109, beschriebenen Arbeitsweisen erhältlich. Die Umwandlurg dieser Aniline in Isocyanate der Formel IV kann mit Phosgen nach allgemein üblichen Verfahren erfolgen. Die Benzoylisocyanate der Formel III, bzw. deren Herstellungsverfahren sind aus der deutschen Offenlegungsschrift Nr. 2 123 236 (vgl. auch J. Org. Chem. *30*, 4306; 1965) bekannt. In Beilstein "Handbuch der organischen Chemie", *Bd. 9*, 336, wird die Herstellung der Benzamide der Formel V beschrieben.

Aus J. Agr. Food Chem. *21* (1973), Nr. 3, S.348—354, sind bestimmte substituierte N-Phenyl-N'-2,6,-dichlorbenzoylharnstoffe mit insektiziden Eigenschaften bekannt. Ferner werden in der deutschen Offenlegungsschrift 26 01 780 substituierte N-Halogenalkyloxyphenyl-N'-phenyl-harnstoffe als insektizide Wirkstoffe beschrieben.

Uberraschenderweise wurde nun gefunden, dass die erfindungsgemässen Verbindungen der Formel I bei guter Pflanzen-verträglichkeit und geringer Warmblütertoxizitat ausgezeichnete Wirksamkeit als Schädlingsbekämpfungsmittel, insbesondere als Insektizide, aufweisen.

Die Verbindungen der Formel I weisen eine breite biozide Wirkung auf und eignen sich zur Bekämpfung von verschiedenartigen, Pflanzen und Tiere befallenden Schadinsekten. Die Verbindungen der Formel I zeichnen sich darüber hinaus durch ihre frasshemmende Wirkung gegenüber pflanzenschädigenden Insekten aus. Insbesondere eignen sich die Verbindungen der Formel I zur Bekämpfung von Insekten der Familien: Acrididae, Blattidae, Gryllidae, Gryllotalpidae, Tettigoniidae, Cimicidae, Pyrrhocoridae, Reduviidae, Aphididae, Delphacidae, Diaspididae, Pseudococcidae, Chrysomelidae, Coccinellidae, Bruchidae, Scarabaeidae, Dermestidae, Tenebrionidae, Curculionidae, Tineidae, Noctuidae, Lymantriidae, Pyralidae, Galleridae, Culicidae, Tipulidae, Stomoxydae, Muscidae, Calliphoridae, Trypetidae und Pulicidae.

Neben ihrer günstigen Wirkung gegenüber Fliegen, wie z.B. Musca domestica, und Mücken können die Verbindungen der Formel I auch zur Bekämpfung von pflanzenschädigenden Insekten, insbesondere von pflanzenschädigenden Frassinsekten, in Zier- und Nutzpflanzen, insbesondere in Baumwollkulturen (z.B. Spodoptera littoralis und Heliothis virescens) und in Gemüsekulturen (z.B. Leptinotarsa decemlineata und Myzus persicae) eingesetzt werden.

Die insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Mit besonderem Vorteil können Verbindungen der Formel I auch mit Substanzen kombiniert werden, welche einen ihre Wirkung verstärkenden Effekt ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan, S,S,S-Tributylphosphorotrithioate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmunger in üblicher Formulierung, die in der Applikationstechnik zum Allgemeinwissen gehören, verarbeitet werden. Ferner sind "cattle dips",

d.h. Viehbäder, und "spray races", d.h. Sprühgänge, in denen wässrige Zubereitungen verwendet werden, zu erwähren.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:
a) in Wasser dispergierbare Wirkstoffkonzentrate: Spritzpulver (wettable powders), Pasten, Emulsionen;
b) Lösungen.

Der Gehalt an Wirkstoff in den oben beschriebenen Mitteln liegt zwischen 0,1 bis 95%.

Die Wirkstoffe der Formel I können beispielsweise wie folgt formuliert werden:

*Stäubemittel:* Zur Herstellung eines a) 5%igen und b) 2%igen Staübemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

*Granulat:* Zur Herstellung eines 5%igen Granulates werden die folgenden Stoffe verwendet:
5 Teile Wirkstoff,
0,25 Teile Epichlorhydrin,
0,25 Teile Cetylpolyglykoläther,
3,50 Teile Polyäthylenglykol,
91 Teile Kaolin (Korngrösse 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

*Spritzpulver:* Zur Herstellung eines a) 40%igen, b) und c) 25%igen d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsaltz,
54 Teile Kieselsäure,

b) 25 Teile Wirkstoff,
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25 Teile Wirkstoff,
2,5 Teile Isooctylphenoxy-polyoxyäthylen-äthanol,
1,7 Teile Champagne-Kreide/Hydroxyäthylcellulose-Gemisch (1:1)
8,3 Teile Natrimaluminiumsilikat,
16,5 Teile Kieselgur,
46 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf

enstprechenden Mühlen und Walzen vermahlen. Man erhält Spritzpulver, die sich mit Wasser zu Suspensionen jeder gewünschten Konzentration verdünnen lassen.

*Emulgierbare Konzentrate:* Zur Herstellung eines a) 10%igen b) 25%igen und c) 50%igen emulgierbaren Konzentrates werden folgende Stoffe verwendet:

a) 10 Teile Wirkstoff
3,4 Teile epoxydiertes Pflanzenöl,
3,4 Teile eines Kombinationsemulgators, bestehend aus Fettalkoholpolyglykoläther und Alkyl-aralkyl-sulfonat-Calcium-Salz,
40 Teile Dimethylformamid,
43,2 Teile Xylol;

b) 25 Teile Wirkstoff,
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol.

c) 50 Teile Wirkstoff,
4,2 Teile Tributylphenol-Polyglykoläther,
5,8 Teil Calcium-Dodecylbenzolsulfonat,
20 Teile Cyclohexanon,
20 Teile Xylol.

Aus solchen Konzentrationen können durch Verdünnen mit Wasser Emulsionen jeder gewünschten Konzentration hergestellt werden.

*Sprühmittel:* Zur Herstellung eines a) 5%igen und b) 95%igen Sprühmittels werden die folgenden Bestandteile verwendet:

a) 5 Teile Wirkstoff,
1 Teil Epichlorhydrin,
94 Teile Benzin (Siedegrenzen 160—190°C);

b) 95 Teile Wirkstoff,
5 Teile Epichlorhydrin.

Beispiel 1

Herstellung von N - 3,5 - Dichlor - 4 - allyloxyphenyl - N' - 2,6 - dichlorbenzoylharnstoff

Zu einer Lösung von 3,3 g 3,5 - Dichlor - 4 - allyloxianilin in 50 ml absolutem Aether werden bei Raumtemperatur 3,5 g 2,6 - Dichlorbenzoylisocyanat in 10 ml absolutem Aether zugetropft.

Der nach kurzer Zeit ausfallende Niederschlag wird abgesaugt und mit Aether gewaschen. Man erhält die Titel-Verbindung der Formel

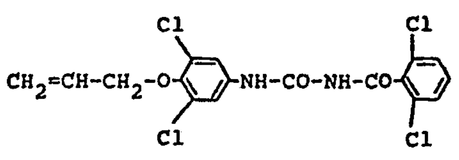

mit einem Schmelzpunkt von 210—211°C.

In analoger Weise werden auch die folgenden Verbindungen hergestellt:

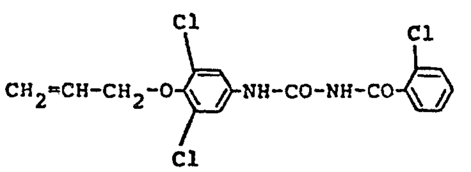

Smp.: 174—175°C

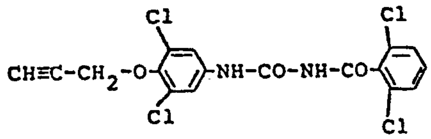

Smp.: 197—198°C

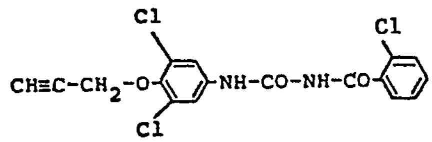

Smp.: 224—226°C

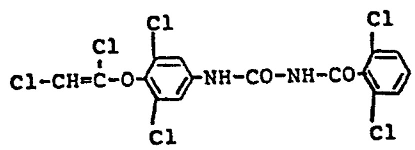

Smp.: 232—234°C

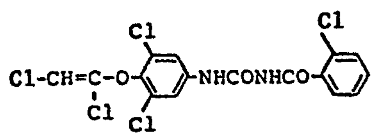

Smp.: 199—200°C

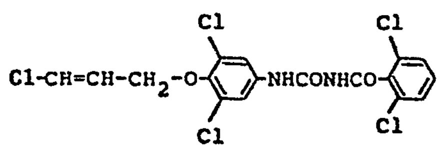

Smp.: 163—164°C

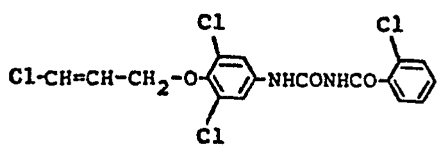

Smp.: 172—173°C

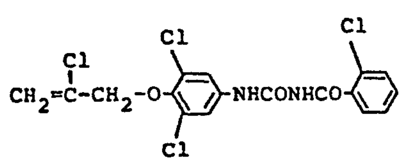

Smp.: 195—196°C

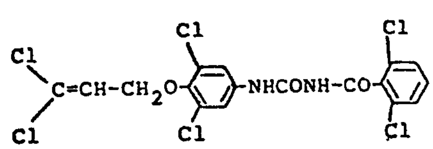

Smp.: 171—172°C

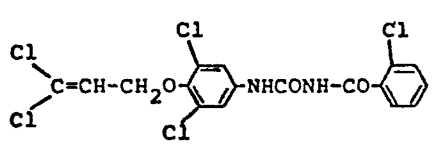

Smp.: 201—203°C

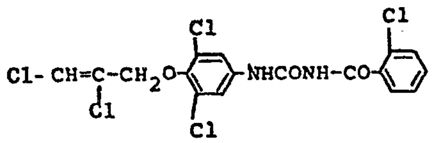

Fp.: 202—205°C

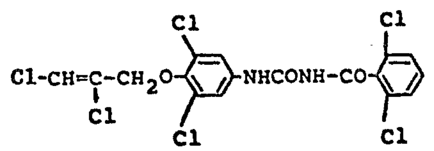

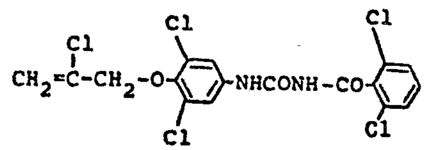

Beispiel 2

*Wirkung gegen Musca domestica*

je 50 g Madensubstrat wurden in Becher abgewogen. Von einer 1%igen acetonischen Lösung wurden pro Aktivsubstanz zwiemal je 2,5 ml auf 50 g Madensubstrat pipettiert. Nach dem Durchmischen des behandelten Substrates lässt man das Lösungsmittel abdampfen. Pro Wirkstoff wurden dann je 25 1-, 2- und 3-tägige Maden und ca.50 Fliegeneier angesetzt. Nach Abschluss der Verpuppung wurden die Puppen ausgeschwemmt und gezählt. Nach 10 Tagen wurde die Anzähl geschlüpfter Fliegen bestimmt und damit ein allfälliger Einfluss auf die Metamorphose festgestellt. Verbindungen gemäss Beispiel 1 zeigten in diesem Test eine gute Wirksamkeit.

Beispiel 3

*Wirkung gegen Lucilia sericata*

Zu 9 ml eines Zuchtmediums wurden bei 50°C 1 ml einer 0,5% Aktivsubstanz enthaltenden wässrigen Lösung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss Beispiel 1 zeigten in diesem Test eine gute Wirkung gegen Lucilia sericata.

Beispiel 4

*Wirkung gegen Aëdes aegypti*

Auf die Oberfläche von 150 ml Wasser, das sich in einem Behälter befindet, wurde so viel einer 0,1%igen acetonischen Lösung des Wirkstoffs pipettiert, dass Konzentrationen von je 10, 5 und 1 ppm erhalten wurden. Nach Verdunsten des Acetons wurden die Behälter mit 30—40 2-tägigen Aëdes-Larven beschickt. Nach 1, 2 und 5 Tagen wurde die Mortalität geprüft.

Verbindungen gemäss Beispiel 1 zeigten in diesem Test eine gute Wirkung gegen Aëdes aegypti.

Beispiel 5

*Insektizide Frassgift-Wirkung*

Baumwollpflanzen wurden mit einer 0,05%igen wässrigen wirkstoffemulsion (erhalten aus einem 10%igen emulgierbaren Konzentrat)besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

**Patentansprüche**

1. N - Phenyl - N' - benzoylharnstoff der Formel I

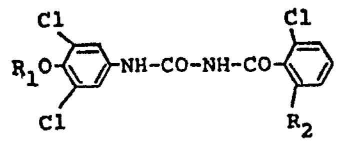

(I)

worin $R_1$ $CH_2{=}CH{-}CH_2{-}$, $CHCl{=}CCl{-}$, $CHCl{=}CH{-}CH_2{-}$, $CH_2{=}CCl{-}CH_2{-}$, $CCl_2{=}CH{-}CH_2{-}$, $CHCl{=}CCl{-}CH_2{-}$ oder $CH{\equiv}C{-}CH_2{-}$ und

$R_2$ Wasserstoff oder Chlor bedeuten.

2. Verbindung gemäss Anspruch 1 der Formel I, dadurch gekennzeichnet, dass $R_2$ Wasserstoff bedeutet.

3. Verbindung gemäss Anspruch 1 der Formel I, dadurch gekennzeichnet, dass $R_1$ $CH_2{=}CH{-}CH_2{-}$, $CHCl{=}CCl{-}$ oder $CHCl{=}CH{-}CH_2{-}$ bedeutet.

4. Verbindung gemäss Anspruch 2, dadurch gekennzeichnet, dass $R_2$ Wasserstoff bedeutet.

5. Verbindung gemäss Anspruch 4 der Formel

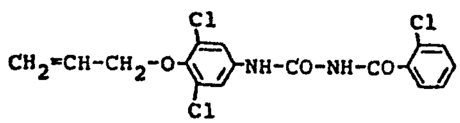

6. Verbindung gemäss Anspruch 4 der Formel

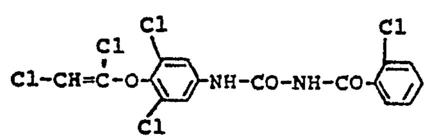

7. Verbindung gemäss Anspruch 4 der Formel

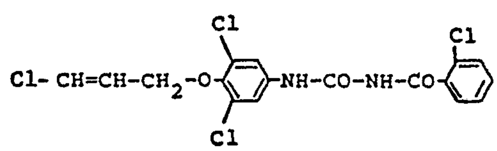

8. Verfahren zur Herstellung von Verbindungen der Formel I gemäss Anspruch 1, dadurch gekennzeichnet, dass man

a) eine Verbindung der Formel II

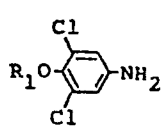

(II)

mit einer Verbindung der Formel III

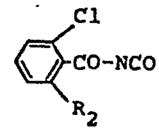

(III)

oder

b) eine Verbindung der Formel IV

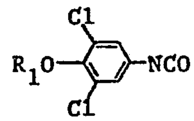

(IV)

mit einer Verbindung der Formel V

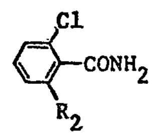

(V)

umsetzt, wobei $R_1$ und $R_2$ die im Anspruch 1 angegebenen Bedeutungen haben.

9. Schädlingsbekämpfungsmittel, welches als aktive Komponente eine Verbindung der Formel I gemäss einem der Ansprüche 1 bis 7 und geeignete Trager und/oder andere Zuschlagstoffe enthält.

10. Verwendung einer Verbindung der Formel I gemäss einem der Ansprüche 1 bis 7 zur Bekämpfung von Pflanzen befallenden Schädlingen.

## Claims

1. N-Phenyl-N'-benzoylurea of the formula I

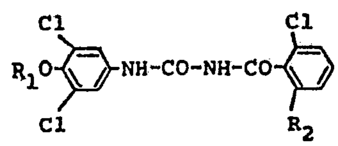

(I)

wherein

$R_1$ represents $CH_2{=}CH{-}CH_2{-}$, $CHCl{=}CCl{-}$, $CHCl{=}CH{-}CH_2{-}$, $CH_2{=}CCl{-}CH_2{-}$, $CCl_2{=}CH{-}CH_2{-}$, $CHCl{=}CCl{-}CH_2{-}$ or $CH{\equiv}C{-}CH_2{-}$, and

$R_2$ represents hydrogen or chlorine.

2. A compound according to Claim 1 of the formula I, characterised in that $R_2$ represents hydrogen.

3. A compound according to Claim 1 of the formula I, characterised in that $R_1$ represents $CH_2{=}CH{-}CH_2{-}$, $CHCl{=}CCl{-}$ or $CHCl{=}CH{-}CH_2{-}$.

4. A compound according to Claim 2, characterised in that $R_2$ represents hydrogen.

5. A compound according to Claim 4 of the formula

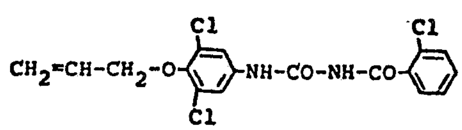

6. A compound according to Claim 4 of the formula

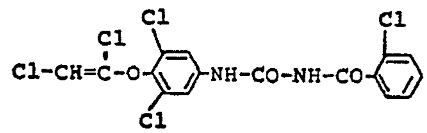

7. A compound according to Claim 4 of the formula

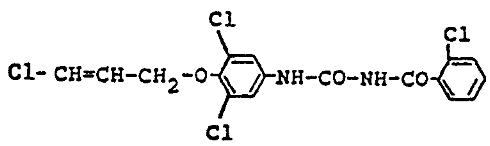

8. A process for producing compounds of the formula I according to Claim 1, characterised in that a) a compound of the formula II

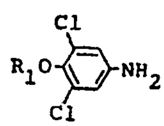

(II)

is reacted with a compound of the formula III

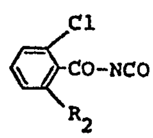

(III),

or

b) a compound of the formula IV

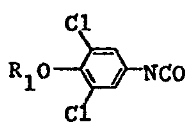

(IV)

is reacted with a compound of the formula V

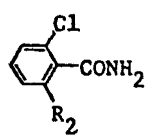

(V),

wherein $R_1$ and $R_2$ have the meanings given in Claim 1.

9. A pesticidal composition which comprises a compound of the formula I according to any one of Claims 1 to 7 as active ingredient, and suitable carriers and/or other additives.

10. Use of a compound of the formula I according to any one of Claims 1 to 7 for combating pests which infest plants.

**Revendications**

1. N-phényl-N'-benzoylurée de formule I

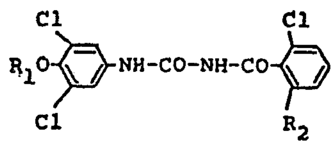

(I)

dans laquelle $R_1$ représente

$CH_2{=}CH{-\!-}CH_2{-\!-}$, $CHCl{=}CCl{-\!-}$, $CHCl{-\!-}CH{-\!-}CH_2$, $CH_2{=}CCl{-\!-}CH_2{-\!-}$, $CCl_2{=}CH{-\!-}CH_2{-\!-}$, $CHCl{=}CCL{-\!-}CH_2{-\!-}$ ou $CH{=}C{-\!-}CH_2{-\!-}$ et $R_2$ représente l'hydrogène ou le chlore.

2. Composé selon la revendication 1, répondant à la formule I, caractérisé en ce que $R_2$ représente l'hydrogène.

3. Composé selon la revendication 1, répondant à la formule I, caractérisé en ce que $R_1$ représente $CH_2{=}CH{-\!-}CH_2{-\!-}$, $CHCl{=}CCl{-\!-}$ ou $CHCl{=}CH{-\!-}CH_2{-\!-}$.

4. Composé selon la revendication 2, caractérisé en ce que $R_2$ représente l'hydrogène.

5. Composé selon la revendication 4, répondant à la formule

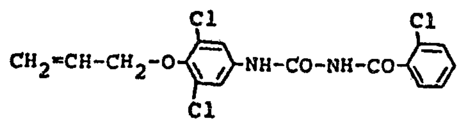

6. Composé selon la revendication 4, répondant à la formule

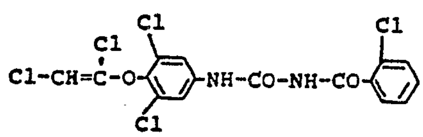

7. Composé selon la revendication 4, répondant à la formule

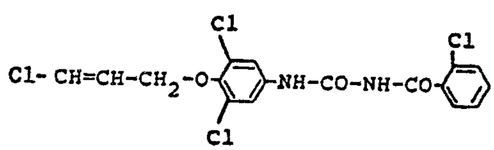

8. Procédé pour préparer les composés de formule I selon la revendication 1, caractérisé en ce que

a) on fait réagir un composé de formule II

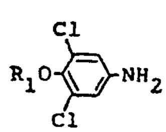

(II)

avec un composé de formule III

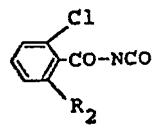

(III)

ou bien

b) on fait réagir un composé de formule IV

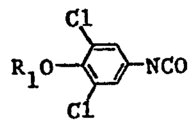

(IV)

avec un composé de formule V

(V)

$R_1$ et $R_2$ ayant les significations indiquées dans la revendication 1.

9. Pesticide contenant, en tant que composant actif, un composé de formule I selon l'une des revendications 1 à 7 et des véhicules et/ou autres additifs appropriés.

10. Utilisation d'un composé de formule I selon l'une des revendications 1 à 7 pour la lutte contre les parasites des végétaux.